# EUROPÄISCHE PATENTANMELDUNG

(11) **EP 0 911 388 A2**
(43) Veröffentlichungstag der Anmeldung: **28.04.1999**
(21) Anmeldenummer: 98111599.1
(22) Anmeldetag: 24.06.1998
(51) Int. Cl.: C12N 1/36, C12N 5/06, C12N 5/10

(54) **Verfahren zur Stimulation von T-Zellen mit gewünschter Antigenspezifität**

(30) Priorität: 15.09.1997 DE 19740571
(71) Anmelder: GSF-Forschungszentrum für Umwelt und Gesundheit, GmbH, 85764 Oberschleissheim (DE)
(72) Erfinder: Staege, Martin S., Dr., 80689 München (DE); Kempkes, Bettina, Dr., 80678 München (DE); Bornkamm, Georg W., Prof. Dr., 81243 München (DE); Hammerschmidt, Wolfgang, Dr., 81247 München (DE); Zimber-Strobl, Ursula, Dr., 82110 Germering (DE); Polack, Axel, Dr., 80798 München (DE)
(74) Vertreter: Reinhard - Skuhra - Weise & Partner

(57) **Zusammenfassung**

Die Erfindung beschreibt Verfahren zur Stimulation von T-Zellen mit gewünschter Antigenspezifität, wobei ein bevorzugtes Verfahren die nachfolgenden Schritte aufweist:
(a) Einführen von Immortalisierungs-Genen in antigenpräsentierende Zellen (APC) in solcher Weise, daß zumindest die Expression und/oder Funktion zumindest eines dieser Gene regulierbar ist, um konditional immortalisierte antigenpräsentierende Zellen zu erhalten;
(b) Einführen eines für das gewünschte Antigen kodierenden Gens in die im Schritt (a) erhaltenen immortalisierten Zellen in solcher Weise, daß das Antigen zumindest auch nach dem Stop der Expression und/oder der Aufhebung der Funktion zumindest eines der Immortalisierungsgene exprimierbar ist;
(c) Expansion der immortalisierten antigenpräsentierenden Zellen durch Expression der Immortalisierungsgene und/oder durch funktionelle Aktivierung der Immortalisierungsgene;
(d) Beendigung der Proliferation der immortalisierten antigenpräsentierenden Zellen durch Stop der Expression und/oder durch Aufhebung der Funktion zumindest eines der regulierbaren Immortalisierungsgene;
(e) fortgesetzte Expression des Antigens;
(f) Zugabe leukozytärer Zellen, die zumindest T-Zellen enthalten, und Kultivierung des Zellgemisches zur Stimulation der gegen das gewünschte Antigen gerichteten T-Zellen;
(g) wahlweise Aufreinigung und Isolierung der stimulierten T-Zellen.

## Beschreibung

Die vorliegende Erfindung betrifft Verfahren zur Stimulation von T-Zellen mit gewünschter Antigenspezifität.

Die verschiedenen Subpopulationen von T-Zellen spielen einerseits bei der Regulation immunologischer Prozesse, andererseits als Effektorzellen der zellulären Zytotoxizität eine bedeutende Rolle bei der Abwehr von Infektionskrankheiten und bösartigen Neubildungen sowie bei der Entstehung von Autoimmunitätserkrankungen. Daher ist die Modulation der T-Zellaktivität ein zentrales Anliegen im Rahmen der Therapie zahlreicher Krankheiten. Unter "Stimulation" wird erfindungsgemäß nicht nur eine Verstärkung einer bereits bestehenden T-Zell vermittelten Immunantwort verstanden, sondern auch deren Induktion, um die Abwehr von Pathogenen oder Tumorzellen zu erzielen; weiterhin besteht bei Autoimmunkrankheiten und Allergien der Wunsch, die ungewünschte Immunantwort zu unterdrücken.

Zur Induktion einer T-Zellantwort wurden in der Vergangenheit verschiedene Wege beschritten. Zum einen werden das native Antigen [2] oder antigene Peptide [3,4] *in-vivo* und *in-vitro* zur Induktion einer T-Zellantwort eingesetzt, zum anderen wird das Gen für das entsprechende Antigen durch Gentransfer in geeignete Zellen eingeführt und die derart veränderten Zellen als antigenpräsentierende Zellen (APC) verwendet. Wegen ihrer hervorragenden immunstimulatorischen Eigenschaften finden hierbei insbesondere dendritische Zellen (DC) Verwendung [5].

Jede dieser Methoden hat unterschiedliche Vor- und Nachteile. So ist der Einsatz von nativen Antigenen nicht geeignet, um zytotoxische T-Zellen (CTL) zu induzieren. CTL erkennen das relevante antigene Peptid i.a. in Kombination mit Klasse-I-Molekülen des Haupthistokompatibilitätskomplexes (MHC). Die in Kombination mit Klasse-I-MHC-Molekülen präsentierten Peptide stammen jedoch in der Regel aus der intrazytoplasmatischen Prozessierung des Antigens. Da exogen angebotene Antigene nicht in den Klasse-I-Weg der Antigenprozessierung gelangen, ist es im allgemeinen nicht möglich, durch Immunisierung mit nativen Antigenen CTL zu induzieren.

Peptide bieten demgegenüber den Vorteil, extern an MHC-Moleküle binden zu können, ohne das Zytoplasma der APC erreichen zu müssen. Der Einsatz von Peptiden zur T-Zellgenerierung bzw. T-Zell-Stimulation setzt jedoch voraus, daß die antigenen Determinanten (Epitope) der einzelnen Antigene bekannt sind. Da MHC-Moleküle nur in der Lage sind, Peptide mit konservierten Strukturmerkmalen zu binden, diese Konsensussequenzen aber vom MHC-Molekül determiniert werden und für jedes Allel anders sind, ergibt sich die Notwendigkeit, für jede Kombination aus MHC-Haplotyp und Antigen die geeigneten Peptide erneut zu bestimmen. Abgesehen davon, daß dies zeit- und arbeitsaufwendig ist, steht die benötigte Technik nicht an jedem Ort zur Verfügung. Darüber hinaus ist der Einsatz von Peptiden zumindest *in-vivo* dadurch fragwürdig, daß neben der Induktion einer spezifischen T-Zellimmunität auch die Induktion einer spezifischen Toleranz möglich [6] ist.

Der Einsatz von antigenpräsentierenden DC zur Immunisierung wird dadurch limitiert, daß Isolierung, Kultur und Gentransfer in diese Zellen schwierig sind und darüberhinaus die Anzahl der verfügbaren DC bei zahlreichen Krankheiten stark eingeschränkt ist.

Aktivierte B-Zellen gehören neben DC zu den potentesten APC, die wir kennen. Interessanterweise führt die Immortalisation von B-Zellen mit Epstein-Barr-Virus zu Zellen (lymphoblastoiden Zell-Linien, LCL), welche den Phänotyp aktivierter B-Zellen repräsentieren [7] . Diese Zellen sind ausgezeichnete APC, haben ein fast unbegrenztes Proliferationspotential und werden daher auch als solche für zahlreiche immunologische Experimente im Humansystem eingesetzt, bei welchen der regelmäßige Zugriff auf andere APC nicht notwendig und/oder aus ethischen Gründen nicht gerechtfertigt ist. Prinzipiell sollten LCL als APC zur Induktion jeder gewünschten T-Zellantwort geeignet sein. Dieses wird jedoch dadurch erschwert, daß diese Zellen auch eine starke EBV-spezifische T-zellvermittelte Immunantwort induzieren, die die gewünschte Immunantwort überlagert [8,9,10]. Da die Durchseuchung der Bevölkerung mit EBV extrem hoch ist und die Frequenz EBV-spezifischer T-Zellen in latent EBV-infizierten Personen zu den höchsten bekannten T-Zellfrequenzen (nur noch erreicht von der Frequenz allospezifischer T-Zellen) zählt, eignen sich EBV-immortalisierte autologe B-Zellen zwar gut zur Restimulation, sind jedoch für die Primärstimulation von T-Zellen, die nicht EBV Antigene erkennen, ungeeignet.

Es ist eine Aufgabe der vorliegenden Erfindung, ein neues Verfahren zur Stimulation von T-Zellen mit gewünschter Antigenspezifität bereitzustellen.

Diese Aufgabe wird erfindungsgemäß durch ein Verfahren (1) gelöst, das zumindest die nachfolgenden Schritte umfaßt:
(a) Einführen von Immortalisierungs-Genen in antigenpräsentierende Zellen (APC) in solcher Weise, daß zumindest die Expression und/oder Funktion zumindest eines dieser Gene regulierbar ist, um konditional immortalisierte antigenpräsentierende Zellen zu erhalten;
(b) Einführen eines für das gewünschte Antigen kodierenden Gens in die im Schritt (a) erhaltenen immortalisierten Zellen in solcher Weise, daß das Antigen zumindest auch nach dem Stop der Expression und/oder der Aufhebung der Funktion zumindest eines der Immortalisierungsgene exprimierbar ist;
(c) Expansion der immortalisierten antigenpräsentierenden Zellen durch Expression der Immortalisierungsgene und/oder durch funktionelle Aktivierung der Immortalisierungsgene;
(d) Beendigung der Proliferation der immortalisierten antigenpräsentierenden Zellen durch Stop der Expression und/oder durch Aufhebung der Funktion zumindest eines der regulierbaren Immortalisierungsgene;
(e) fortgesetzte Expression des Antigens;
(f) Zugabe leukozytärer Zellen, die zumindest T-Zellen enthalten, und Kultivierung des Zellgemisches zur Stimulation der gegen das gewünschte Antigen gerichteten T-Zellen;
(g) wahlweise Aufreinigung und Isolierung der stimulierten T-Zellen.

In einer Ausgestaltung dieses Verfahrens werden im Schritt (a) APC aus einem Spender A zugegeben und im Schritt (f) allogene, leukozytäre Zellen aus einem anderen Spender B, welcher syngen für mindestens ein entsprechendes, der Antigenpräsentation dienendes MHC-Molekül mit dem Spender A der APC ist.

In einer weiteren Ausführungsform können die im Schritt (a) verwendeten antigenpräsentierenden Zellen vom gleichen Spender wie die leukozytären Zellen im Schritt (f) stammen.

Es ist nicht zwingend notwendig, die oben genannten Schritte in der aufgeführten Reihenfolge durchzuführen. Es ist auch möglich, die Reihenfolge dieser Schritte zu variieren, wobei sie weiterhin zu der gewünschten Stimulation von T-Zellen mit der erstrebten Antigenspezifität führen. Beispielsweise kann der Schritt (b) erst unmittelbar vor dem Schritt (e) durchgeführt werden, ohne von der Erfindung abzuweichen.

Nachfolgend wird eine weitere Ausgestaltung des erfindungsgemäßen Verfahrens beschrieben, das die nachfolgenden Schritte umfaßt und ebenfalls zu der angestrebten Stimulation von T-Zellen mit gewünschter Antigenspezifität führt:
(a) Einführen von Immortalisierungs-Genen in antigenpräsentierende Zellen (APC) in solcher Weise, daß zumindest die Expression und/oder Funktion zumindest eines dieser Gene regulierbar ist, um konditional immortalisierte antigenpräsentierende Zellen zu erhalten;
(b) Einführen eines für das gewünschte Antigen kodierenden Gens in die im Schritt (a) erhaltenen immortalisierten Zellen in solcher Weise, daß das Antigen zumindest auch nach dem Stop der Expression und/oder der Aufhebung der Funktion zumindest eines der Immortalisierungsgene exprimierbar ist;
(c) Expansion der immortalisierten antigenpräsentierenden Zellen durch Expression der Immortalisierungsgene und/oder durch funktionelle Aktivierung der Immortalisierungsgene;
(d) Zugabe leukozytärer Zellen, die zumindest T-Zellen enthalten, zu einem ersten Teil (Aliquot) der aus den Schritten (a) bis (c) erhaltenen immortalisierten, das gewünschte Antigen exprimierenden Zellen und Kultivierung des Zellgemisches zur Stimulation der gegen das gewünschte Antigen gerichteten T-Zellen;
(e) Beendigung der Proliferation des zweiten Teils der aus den Schritten (a) bis (c) erhaltenen APC durch Stop der Expression und/oder durch Aufhebung der Funktion zumindest eines der für die Immortalisierung notwendigen regulierbaren Gene;
(f) Ko-Kultivierung der antigenpräsentierenden Zellen nach dem Stop der Expression und/oder der Aufhebung der Funktion zumindest eines der regulierbaren Immortalisierungsgene aus (e) mit den im Schritt (d) stimulierten T-Zellen zur Restimulation der gegen das gewünschte Antigen gerichteten T-Zellen; und
(g) wahlweise Aufreinigung und Isolierung der stimulierten T-Zellen.

Dieses zuletzt genannte Verfahren wird als Verfahren Nr. 2 bezeichnet. Das Verfahren 2 kann wie folgt ausgestaltet werden:

Im Schritt (a) werden APC aus einem Spender A zugegeben und im Schritt (d) allogene, leukozytäre Zellen aus einem anderen Spender B, welcher syngen für mindestens ein entsprechendes, der Antigenpräsentation dienendes MHC-Molekül mit dem Spender A der APC ist.

In einer bevorzugten Ausgestaltung der Erfindung stammen die im Schritt (a) verwendeten APC des Verfahrens Hr. 2 vom gleichen Spender wie die leukozytären Zellen im Schritt (d).

Wie bereits für das Verfahren 1 beschrieben, ist es auch bei der Verfahrensführung Nr. 2 nicht zwingend notwendig, die Verfahrensschritte (a) bis (g) in der angegebenen Reihenfolge durchzuführen, sondern für den Fachmann sind auch Abwandlungen ersichtlich, die ebenfalls zur gewünschten Stimulation antigenspezifischer T-Zellen führen. Beispielsweise kann der Schritt (b) erst unmittelbar vor dem Schritt (d) oder der Schritt (b) je unmittelbar vor den Schritten (d) und (f) erfolgen.

Wie oben bereits ausgeführt, umfaßt der in den Ansprüchen verwendete Ausdruck "Stimulation" nicht nur eine Induktion einer T-Zell-vermittelten Immunantwort, sondern auch eine Verstärkung einer bereits bestehenden T-Zell vermittelten Immunantwort oder die Induktion einer Anergie in den stimulierten T-Zellen.

Erfindungsgemäß werden antigenpräsentierende Zellen, nachfolgend kurz als "APC" bezeichnet, konditional immortalisiert, so daß zumindest die Expression und/oder die Funktion eines der zur Immortalisierung notwendigen Gene regulierbar ist. Als APC können beliebige, dem Fachmann bekannte antigenpräsentierende Zellen eingesetzt werden, die befähigt sind, ein Antigen so zu präsentieren, daß es von T-Zellen erkannt wird. Bevorzugt können als antigenpräsentierende Zellen B-Zellen, Makrophagen, dendritische Zellen und/oder Fibroblastenzellen eingesetzt werden, wobei B-Zellen besonders bevorzugt sind.

Unter "Immortalisierungsgene" werden erfindungsgemäß solche Gene verstanden, die notwendig sind, um die antigenpräsentierenden Zellen unsterblich zu machen, d.h. deren funktionelle Expression in geeigneten Zellen entweder alleine oder in Kombination mit anderen Faktoren zur unbeschränkten Proliferation dieser Zellen führt.
Bevorzugt werden B-Zellen eingesetzt, die immortalisiert wurden, so daß deren antigenpräsentierenden Eigenschaften mit der beliebigen Expandierbarkeit dieser Zellen in Kultur vereint wird. Die immortalisierten, antigenpräsentierenden Zellen, die von beliebigen Spendern stammen können, stehen damit in unbegrenzter Menge für die primäre und sekundäre Stimulation von T-Zellen zur Verfügung.

Die zur Immortalisierung notwendigen Gene stammen beispielsweise aus dem Epstein-Barr Virus (EBV) oder aus Adenoviren; es können aber zur Immortalisierung auch Oncogene eingesetzt werden. Die jeweils mindestens notwendigen Gene zur Immortalisierung der APC sind entweder in der Literatur beschrieben oder können, ausgehend vom vollständigen Genom beispielsweise eines Virus, durch Deletionen erhalten werden. Die Immortalisierungsgene des EBV sind beispielsweise auf mini-EBV-Vektoren vorhanden, die bspw. in [11] beschrieben sind. Zur vollständigen Offenbarung wird auf diese Literaturstelle voll inhaltlich Bezug genommen.

Die Gene werden besonders bevorzugt aus den Immortalisierungsgenen des EBV ausgewählt, beispielsweise EBNA2-, EBNA3a-, EBNA3b-EBNA3c- oder LMP-Gene.

Neben EBV sind z.B. Adenoviren geeignet, um konditional immortalisierte APC zu generieren. Von Spitkovsky et al. (J.Virol. 1994, 68(4): 2206) wurde gezeigt, daß es möglich ist, Zellen mit dem adenoviralen E1A-Gen konditional zu immortalisieren, wenn dieses Gen an die Hormonbindungs-Domäne des Östrogen-Rezeptors fusioniert ist und gleichzeitig c-ras in den Zellen exprimiert wird. Zwar wurden von den Autoren Fibroblasten als Zielzellen verwendet, es wurde aber bereits vor Jahren gezeigt, daß Fibroblasten durchaus als APC dienen können, wenn man sie mit CD54 und MHC-Molekülen transfiziert. Somit wäre es denkbar, Fibroblasten mit Vektoren zu transfizieren, welche das E1A konditional sowie z.B. CD54, HLA-A2.1 und cdk-4^{R23C} (ein Tumorantigen, welches in Kombination mit HLA-A2 präsentiert wird und in diesem Falle das gewünschte Antigen darstellen würde) enthalten, in Anwesenheit der E1A-Funktion zu expandieren und anschließend nach Abschalten der E1A-Funktion als APC für entsprechende leukozytäre Zellen einzusetzen.

Um die antigenpräsentierenden Zellen zu immortalisieren, können auch transformierende Gene wie Oncogene verwendet werden. Beispielsweise führt die gleichzeitige Überexpression von c-myc und eines anderen Oncogens wie ras oder abl zur Transformation von Zellen. Beispielsweise könnte eines der Oncogene wie das c-myc-Oncogen durch ein Antibiotikum wie Tetracyclin regulierbar gemacht werden, so daß die konditional immortalisierten, antigenpräsentierenden Zellen nur in Abwesenheit des Antibiotikums, d.h. in Anwesenheit der myc-Expression, proliferieren.

Erfindungsgemäß ist zumindest eines der zur Immortalisierung notwendigen Gene, beispielsweise des EBV, in regulierbarer Form vorhanden. "Regulation" bedeutet, daß die Expression dieses Gens oder die Funktion dieses Gens so regulierbar ist, daß durch An- oder Abschalten dieses Gens oder der Funktion seines Proteins die Proliferation der antigenpräsentierenden Zellen beendet werden kann.

Neben der Möglichkeit, die Expression eines zur Immortalisierung notwendigen Genes regulierbar zu gestalten, besteht die Möglichkeit, das entsprechende Gen konstitutiv zu exprimieren, jedoch in einer Form, in der die Funktion des entstehenden Proteins z. B. durch Hormone regulierbar ist. Zum Beispiel lässt sich dadurch, daß man ein Fusionsprotein aus der Hormonbindungsstelle des Östrogenrezeptors und dem gewünschen immortalisierenden Agens konstitutiv exprimiert, erreichen, daß nur nach Bindung von Östrogen an den Rezeptorteil des Fusionsproteins dasselbige in den Kern transloziert wird und dort die Funktion des immortalisierenden Anteils des Fusionsproteins aktiv ist.

Als das zur Immortalisierung notwendige regulierbare Gen des EBV können beispielsweise verwendet werden: das EBNA2-Gen, Das EBNA3a-Gen, das EBNA3b-Gen, das EBNA3c-Gen oder das LMP-Gen.

Die Regulation des regulierbaren Immortalisierungs-Gens kann durch beliebige, aus dem Stand der Technik bekannte Methoden erfolgen. Beispielsweise kann vor die für das Gen kodierende Transkriptionseinheit eine Hormonbindungsstelle einkloniert werden, so daß das Genprodukt in Anwesenheit des Hormons funktionell und in Abwesenheit des Hormons nicht funktionell ist. Außer Hormonen sind beispielsweise auch Antibiotikum-Bindungsstellen verwendbar. Als Hormon-Bindungsstelle kann beispielsweise eine Androgen- oder Östrogenbindungsstelle verwendet werden.

In einer bevorzugten Ausführungsform liegt das für das gewünschte Antigen kodierende Gen auf dem gleichen Vektor, der die zur Immortalisierung notwendigen Gene trägt, wobei wie oben ausgeführt, zumindest eines dieser zur Immortalisierung notwendigen Gene in regulierbarer Form vorliegen muß.

Das Einführen der zur Immortalisierung notwendigen Gene des EBV und das Einführen eines für das gewünschte Antigen kodierenden Gens können entweder getrennt voneinander oder in einem einzigen Schritt erfolgen, wobei bevorzugt die zur Immortalisierung notwendigen Gene und das für das gewünschte Antigen kodierende Gen auf einem Vektor angeordnet werden.

Als Vektoren zum Einführen des für das gewünschte Antigen kodierenden Gens können beliebige, aus dem Stand der Technik bekannte Vektoren verwendet werden, die geeignet sind, das gewünschte Antigen in der immortalisierten, antigenpräsentierenden Zeile zu exprimieren. Die vorliegende Erfindung wird nachfolgend am Beispiel eines Antigens beschrieben, das mit einem von Vaccinia abgeleiteten Vektor in die Zelle eingeführt wurde. Es sind jedoch auch andere Vektoren anwendbar, beispielsweise von EBV, Adenoviren, von Retroviren, von Foamyviren, anderen Poxviren als Vaccinia oder von SV40 abgeleitete Vektoren.

In die Vektoren können beliebige Antigene einkloniert und mit Hilfe dieser Vektoren in die APC eingeführt werden. Mögliche Antigene sind, hier nur beispielhaft aufgezählt: virale Antigene, beispielsweise das pp65 des humanen Zytomegalievirus, oder das EBNA3b des EBV; Tumorantigene, beispielsweise p16^{INK4a}-insensitive Mutante der cdk4 oder das bcr-abl-Fusionsprotein; bakterielle Antigene, beispielsweise Listeriolysin oder Streptolysin oder experimentell verwendete Modelantigene wie Ovalbumin oder Hühnereiweißlysozym.

Die Expansion der immortalisierten APC erfolgt unter an sich bekannten Bedingungen, die in Abhängigkeit von der verwendeten antigenpräsentierenden Zelle eingestellt werden.

Nachdem das für das gewünschte Antigen kodierende Gen und die zur Immortalisierung notwendigen Gene des EBV in die antigenpräsentierende Zellen eingeführt wurden, werden diese expandiert, bis eine ausreichend hohe Zellzahl vorliegt. Durch Stop der Expression zumindest eines der für die Immortalisierung notwendigen regulierbaren Gene oder durch Aufhebung seiner Funktion werden die Proliferation und damit die Expansion der antigenpräsentierenden immortalisierten Zellen gestoppt. Die Expression des Antigens wird jedoch fortgesetzt, da diese durch den Stop der Proliferation nicht beeinflußt wird.

Nach Beendigung der Proliferation der immortalisierten, antigenpräsentierenden Zellen werden leukozytäre Zellen, die zumindest T-Zellen enthalten, zugegeben, und dieses Zellgemisch zur Stimulation der gegen das gewünschte Antigen gerichteten T-Zellen kultiviert.

Leukozytäre Zellen im Sinne dieser Arbeit sind solche, die z.B. aus dem peripheren Blut eines Spenders, beispielsweise durch Dichtegradientenzentrifugation, besonders bevorzugt bei einer Dichte von 1,077, eingestellt beispielsweise mit Ficoll, angereichert werden können. Diese Präparationen enthalten neben Monozyten und NK-Zellen vor allem Lymphozyten, wobei T-Zellen überwiegen. Hierunter fallen auch periphere, mononukleäre Zellen (PBMC), die zumindest T-Zellen enthalten.

Die Kultivierung dieser Zellen zusammen mit den antigenpräsentierenden Zellen erfolgt unter an sich bekannten Kultivierungsbedingungen. Bevorzugte Kultivierungsbedingungen sind in Iscovs modifiziertem Dulbeccos Medium (IMDM) oder RPMI1640 mit den üblichen Zusätzen wie Antibiotika, Interleukin-2 und Serum, in Abhängigkeit vom verwendeten Medium. Die Kultur erfolgt über einen Zeitraum, welcher für das erstrebte Ziel notwendig ist, im allgemeinen für 7 bis 40 Tage. Die induzierten T-Zellen können durch an sich bekannte Methoden aufgereinigt und isoliert werden. Ein Beispiel hierfür ist die Verwendung von Antigkörpern, die an magnetische Kügelchen gekoppelt sind.

Der in der T-Zell-Kultur geübte Experimentator wird nach Ansetzen der Kultur durch tägliche Kontrolle zumindest in der ersten Woche der Kultur die optimalen Kulturbedingungen (IL-2-Konzentration, Serum-Konzentration, Restimulationshäufigkeit) einstellen. Verwendbar sind beispielsweise die nachfolgenden Medien: RPMI+10%FKS+10U/ml IL-2 (Boehringer, Mannheim) + Pen/strep + Amphotericin B sowie CG-Medium (Vitromex, ein Medium auf der Basis von IMDM) mit 10 U/ml IL-2, 5% FKS + Pen/Strep + Amphotericin B. Die Kultur erfolgt normalerweise für 7 Tage, bevor das Ergebnis des Experiments abgelesen wird oder mit frischem Antigen restimuliert wird.

Durch die erfindungsgemäßen Verfahren werden antigenspezifische, zytotoxische T-Zellen und/oder T-Helferzellen und/oder T-Zellen mit Supressor-Eigenschaften induziert, bspw. Klasse I-MHC-restringierte, zytotoxische T-Zellen und Klasse II-MHC-restringierte T-Helferzellen. Induziert werden können somit antigenspezifische CD8-positive und/oder CD4-postive T-Zellen mit αβ-Zell-Rezeptoren und/oder γδ-T-Zell-Rezeptoren.

Es wird darauf hingewiesen, daß die Einführung des Antigens nicht zwingend vor der Expansion der APC durchgeführt werden muß, sondern auch erst unmittelbar vor der Inkubation mit den leukozytären Zellen, beispielsweise bei Verwendung von Vaccinia-Vektoren, erfolgen kann.

Unter "leukozytäre Zellen" fallen auch periphere, mononukleäre Zellen (PBMC), die zumindest T-Zellen enthalten.

Vor Zugabe der leukozytären Zellen werden bevorzugt die immunstimulatorischen Eigenschaften, der das gewünschte Antigen exprimierenden, immortalisierenden Zellen moduliert. Modulation bedeutet, daß die Fähigkeit der antigenpräsentierenden Zellen, die Aktivierung von T-Zellen zu bewirken, in der gewünschten Weise verändert wird. Hierbei muß nicht zwingend eine Verbesserung der stimulatorischen Fähigkeiten angestrebt werden, wie dies im Falle von B-Zellen (als Basis für die konditional immortalisierten APC), beispielsweise durch Kultur in Anwesenheit eines CD40-Stimulus, erreichbar ist. Durch diese Modulation können beispielsweise auch die immunstimulatorischen Eigenschaften so verändert werden, daß in den T-Zellen eine Anergie induziert wird. Hierfür wäre eine Behandlung der konditional immortalisierten APC mit Antikörpern mit Spezifität für das kostimulatorische Molekül B7-1/2 geeignet.

Zum Beispiel können vor der Ko-Kultur der konditional immortalisierten, antigenpräsentierenden Zellen mit den leukozytären Zellen zur Stimulation der hierin enthaltenen T-Zellen die immortalisierten APC durch Kultur in Anwesenheit eines CD40-Stimulus oder in Anwesenheit von Cytokinen moduliert werden.

Der CD40-Stimulus kann durch Zugabe von CD40-exprimierenden Feederzellen und Co-Kultivierung der immortalisierten, antigenpräsentierenden Zellen und der Feederzellen erhalten werden, oder der CD40-Stimulus kann durch Zugabe eines löslichen CD40-Liganden oder eines anti-CD40-Antikörpers erfolgen.

Die hier verwendeten CD40-Ligand exprimierenden Feeder-Zellen wurden beschrieben in [16].

Die konditional immortalisierten antigenpräsentierenden Zellen haben nach Inaktivierung (entweder Stop der Expression oder Blockierung der Funktion) des für die Immortalisierung notwendigen Gens nur eine kurze Lebensdauer. Diese kann durch Kultur auf einem CD40-Ligand (CD40L) exprimierenden Feederlayer deutlich verlängert werden [13]. Bemerkenswerterweise blieb durch die Behandlung mit CD40L-exprimierenden Feederzellen auch die stimulatorische Kapazität dieser Zellen für allogene PBMC erhalten. Die CD40-exprimierenden Feederzellen werden mit den immortalisierten antigenpräsentierenden Zellen ko-kultiviert. Es ist aber auch möglich, daß der CD40-Stimulus durch Zugabe eines löslichen CD40-Liganden oder eines anti-CD40-Antikörpers erfolgt.

In einer bevorzugten Ausgestaltung der Erfindung wird vor Zugabe der leukozytären Zellen das Wachstum der immortalisierten APC dauerhaft unterdrückt. Diese dauerhafte Supression kann beispielsweise durch Zugabe von Mitomycin C oder durch Bestrahlung erfolgen.

Nachfolgend wird die Erfindung zunächst nochmals zusammengefaßt beschrieben. Dabei wird auf die anliegenden Abbildungen Bezug genommen. Die Abbildungen zeigen:
- Abb. 1:: A1-Zellen, aber nicht EREB2-5-Zellen proliferieren weiter nach Östrogenentzug.
- Abb. 2:: EREB2-5-Zellen werden nur in Anwesenheit von funktionellem EBNA2 durch EBV-spezifische CTL lysiert.
- Abb. 3:: Ko-Kultur mit CD40L-exprimierende Feederzellen erhält die allostimulatorischen Fähigkeiten von EREB2-5-Zellen auch in Abwesenheit von Östrogen.
- Abb. 4:: Die Lyse von EREB2-5-Zellen durch antigenspezifische CTL findet auch in Abwesenheit von Östrogen statt, wenn das relevante Antigen konstitutiv zur Expression gebracht wird.
- Abb. 5:: Die allostimulatorische Kapazität von EREB2-5-Zellen ist abhängig von der Anwesenheit von funktionellem EBNA2.
- Abb. 6:: Die primäre Stimulation von allogenen PBMC durch EREB2-5-Zellen führt nicht zur Induktion einer Immunantwort gegen A1-Zellen.

Ein für die Immortalisierung durch EBV essentieller Faktor ist das EBV nukleäre Antigen 2 (EBNA2). Bekannt sind auch konditional durch EBV immortalisierte B-Zellen, die als LCL bezeichnet werden. Dies kann beispielsweise durch Komplementation der EBNA2-Deletion des P3HR1-Virus mit einem Fusionsprotein aus EBNA2 und der Hormonbindungsdomäne des Östrogenrezeptors erreicht werden. Derartige Konstruktionen sind bekannt und werden beispielsweise in [11] beschrieben. Diese als EREB2-5 bezeichnete Zellen proliferieren in Anwesenheit von Östrogen. In Abwesenheit von Östrogen ist EBNA2 inaktiv, und die Zellen stellen die Proliferation ein. Bekannt ist auch, daß durch Transfektion von EREB2-5-Zellen mit einem aktivierten c-myc-Gen eine von EREB2-5-Zellinie (A1) etabliert werden kann, welche auch in Abwesenheit von Östrogen wächst [12]. Diese Zellen spiegeln viele Eigenschaften, beispielsweise das Wachstum, die Expression von Oberflächen-Antigenen usw. von Burkitt-Lymphom-Zellen, also Zellen, in denen EBV in Form der sogenannten Latenz I persistiert, wider.

Bei den erfindungsgemäß durchgeführten Versuchen wurde zunächst die Immunantwort, welche durch die konditional immortalisierte lymphoblastoide Zellinie (LCL) EREB2-5 und die c-myc-transfizierte Tochterzellinie A1 ausgelöst wird, charakterisiert. Hierbei zeigte sich, daß EREB2-5-Zellen, welche in Anwesenheit von Östrogen kultiviert worden waren, eine starke Immunantwort allogener, peripherer, mononukleärer Zellen (PBMC) induzieren, während A1-Zellen, welche in Abwesenheit von Östrogen kultiviert worden waren, dazu nicht in der Lage sind. Desgleichen wurden A1-Zellen unter diesen Umständen nicht von EBV-spezifischen CTL lysiert, während EREB2-5-Zellen sehr wohl lysiert wurden. Wir stellten uns nun die Frage, ob durch Modulation der EBNA2-Funktion in EREB2-5-Zellen bzw. A1-Zellen die Erkennbarkeit der Zellen durch das Immunsystem gesteuert werden kann.

Durch Wiederherstellen der EBNA2-Funktion in A1-Zellen durch Kultur in Anwesenheit von Östrogen erlangten diese Zellen ihre immunstimulatorische Kapazität wieder. Umgekehrt verloren EREB2-5-Zellen überraschenderweise ihre allostimulatorische Potenz praktisch vollständig nach viertägiger Kultur in Abwesenheit von Östrogen. Die Lyse von EREB2-5-Zellen durch CTL mit Spezifität für das EBNA2-regulierte EBV-Antigen LMP2 war nach Östrogenentzug ebenfalls nicht mehr nachweisbar. Umgekehrt wurden jedoch EREB2-5-Zellen auch in Abwesenheit von Östrogen durch CTL lysiert, wenn das entsprechende Antigen durch Infektion der Zellen mit rekombinanten Vaccinia-Viren zur Expression gebracht wurde oder die MHC-Moleküle exogen mit den relevanten Peptiden beladen wurden. In diesem Fall war die Lyse der Zellen nur geringfügig reduziert.

Als APC verwendeten wir B-Zellen, da die Kultur von APC, welche sich von B-Zellen ableiten (Tumor-Zellinien oder LCL), am einfachsten praktizierbar war. Als Quelle dieser B-Zellen diente Nabelschnurblut eines gesunden Kindes, aus welchem die darin enthaltenen, leukozytären Zellen, hier PBMC, gewonnen wurden. Der HLA-Haplotyp des Spenders der PBMC war (A11, 28; B7,49; Cw7; DRw6,7; DQw1,2). Als Immortalisierungsgene verwendeten wir die entsprechenden Gene des EBV, die B-Zellen wurden ko-infiziert mit dem P3HR1-Virus und einem Mini-EBV, welches das EBNA2-Gen fusioniert an den Östrogenrezeptor zur Expression bringt. Diese Vektoren sind in Kempkes et al. (EMBOJ 14:88) beschrieben. Hierdurch wurde erreicht, daß durch das P3HR1-Virus (ein EBV des Typs II) alle Immortalisierungsgene des EBV in die Zellen eingebracht wurden, mit Ausnahme des EBNA2-Gens, für das eine Deletion in diesem Virusstamm vorliegt. Dieses Gen wurde durch die Fusion an den Östrogenrezeptor in seiner Funktion regulierbar: Nur in Anwesenheit von Östrogen im Kulturmedium ist die EBNA2-Funktion aktiv. Daher proliferieren diese konditional immortalisierten LCL nur in Anwesenheit von Östrogen. Da die Expression wesentlicher Gene des EBV durch EBNA2 gesteuert wird, ist in diesen LCL nach Abschaltung der EBNA2-Funktion auch die Expression anderer EBV-Gene herunterreguliert. Diese Zellen wurden EREB2-5 genannt (für Estrogen-Rezeptor/EBNA2-Klon 5) und in Anwesenheit von Östrogen expandiert. Die Proliferation, als Meßparameter für die Expansion wird in Bild 1 gezeigt. Hier wird auch demonstriert, daß die Proliferation nach Östrogenentzug, sprich Inaktivierung des regulierbaren Immortalisierungs-Genes, unterbleibt.

Als Antigene wurden von uns insbesondere das EBNA3b-Gen des EBV-Typs 1 verwendet. Da sich die Sequenz des EBNA3b-Genes zwischen dem EBV-Typ-II, welcher für die Immortalisierung verwendet wurde (P3HRI), und dem Typ I stark unterscheidet, handelt es sich bei diesem Antigen um ein solches, welches nur nach künstlichem Einführen in die Zellen exprimiert wird. Als Vektor zum Einbringen des Antigens wurden rekombinante Vaccinia-Viren verwendet. Diese Vektoren wurden beschrieben in [17].

Es zeigte sich hierbei, daß EREB2-5-Zellen nach dem Einbringen von EBNA3b mittels Vaccinia-Virus von CTL mit Spezifität für dieses Antigen erfolgreich lysiert wurden. Diese Lyse erfolgte auch nach Abschaltung der EBNA2-Funktion durch Östrogenentzug (Bild 4). Die verwendeten CTL waren syngen für das HLA-A11-Allel mit den als Zielzellen eingesetzten EREB2-5, stammten aber von unverwandten Spendern, so daß sie als (semi-)allogene T-Zellen bezeichnet werden können. Bei den verwendeten T-Zellen handelt es sich um T-Zellen, welche bereits früher durch Ko-Kultur mit EBV-positiven Zellen aus den Spendern der T-Zellen etabliert worden waren. Somit liegt hier eine Restimulation der T-Zellen mit allogenen LCL, die syngen für ein der Antigenpräsentation dienendes HLA-Molekül (in diesem Falle HLA-A11) sind, vor. CTL sind offensichtlich durch die verwendeten LCL gut stimulierbar, es lassen sich aber im Prinzip auch durch bereits geschilderte Abänderungen problemlos auch T-Helferzellen stimulieren. Daß LCL hierzu im Prinzip in der Lage sind, zeigt sich darin, daß sich bei Verwendung von EREB2-5-Zellen zur Stimulation alloreaktiver Zellen neben CD8-positiven (CTL) auch CD4-positive (T-Helferzellen) mittels Immunfluoreszenz in den stimulierten Zellen nachweisen lassen. (Daten nicht gezeigt; die hierfür durchgeführte FACS (Fluorescence activated cell scanner) -Analyse wurde mit Methoden durchgeführt, wie sie in der Literaturstelle 15 beschrieben wurden.) Die Lyse von EREB2-5-Zellen durch CTL mit Spezifität für ein Antigen, welches unter Kontrolle von EBNA2 exprimiert wurde (LMP2), fand nur in Anwesenheit der EBNA2-Funktion statt (Bild 2). Somit konnte gezeigt werden, daß nach Abschaltung der EBNA2-Funktion und damit Aufhebung der Immortalisation die Immunantwort gegen das immortalisierende Agens stark reduziert ist, was letztlich der wesentliche Vorteil der Verwendung konditional immortalisierter Zellen gegenüber klassischen immortalisierten APC für den Einsatz als APC zur T-Zellstimulation ist.

Die weiteren Versuche wurden durchgeführt, indem als APC EREB2-5-Zellen ohne weiteres Einbringen eines Antigens als Stimulatorzel-len für allogene PBMC verwendet werden. Hier dienten die allseits vorhandenen Alloantigene (vor allem der allogene HLA) als Antigen.

Durch die Ko-Kultur von EREB2-5-Zellen mit den PBMC allogener Spender wurden die in diesen PBMC enthaltenen T-Zellen stimuliert und begannen zu proliferieren (Bild 5). Hierbei zeigte sich, daß die Proliferation nach Abschaltung der EBNA2-Funktion deutlich reduziert war. Um die immunstimulatorische Potenz der EREB2-5-Zellen zu restaurieren, wurden diese für 4 Tage nach Abschaltung der EBNA2-Funktion auf einem CD40-Ligand-exprimierenden Fibroblasten-Feederlayer kultiviert und anschließend als APC eingesetzt. Durch diese Behandlung ließ sich die stimulatorische Potenz wiederherstellen (Bild 3). Aus der Beobachtung, daß EREB2-5-Zellen in Anwesenheit der EBNA2-Funktion eine gute stimulatorische Potenz für PBMC (Bild 5) und klonierte T-Zellen (Bild 5) tragen, in Abwesenheit der EBNA2-Funktion jedoch nur für bereits geprimte T-Zellen als APC dienlich sind (Bild 4), folgt, daß als alternative Ausführungsform das erfindungsgemäße Verfahren nach Anspruch 2 durchführbar ist. Die einzelnen Schritte dieses Anspruchs 2 wurden bereits oben beschrieben.

Die Aufreinigung der T-Zellen könnte z.B. mittels FACS erfolgen.

Nachfolgend werden die oben beschriebenen Experimente, die zu den erfindungsgemäßen Verfahren geführt haben, genauer beschrieben.

Als antigenpräsentierende Zellen wurden B-Zellen verwendet. Unter den in die antigenpräsentierende Zellen eingeführten, zur Immortalisierung notwendigen Gene des EBV wurde das EBNA2-Gen durch eine Östrogenbindungsdomäne regulierbar gemacht. Hierbei handelt es sich nur um eine mögliche Ausgestaltung der erfindungsgemäßen Verfahren. Wie oben bereits ausgeführt, sind auch andere Ausgestaltungen möglich, die im Bereich des fachmännischen Könnens eines auf dem immunologischen Gebiet arbeitenden Wissenschaftlers liegen.

### 1. Einsatz konditional EBV-immortalisierter LCL zur T-Zell-Aktivierung nach Abschaltung von EBNA2 und CD40-Stimulation

Bei dieser Ausgestaltung des erfindungsgemäßen Verfahrens werden an einem Spender der LCL konditional immortalisierte LCL generiert und als antigenpräsentierende Zellen eingesetzt. In diesen LCL wird das gewünschte Antigen zur Expression gebracht.

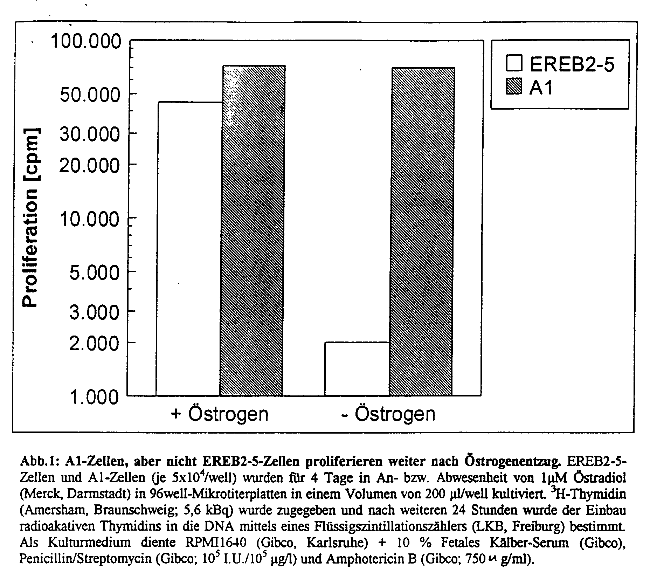

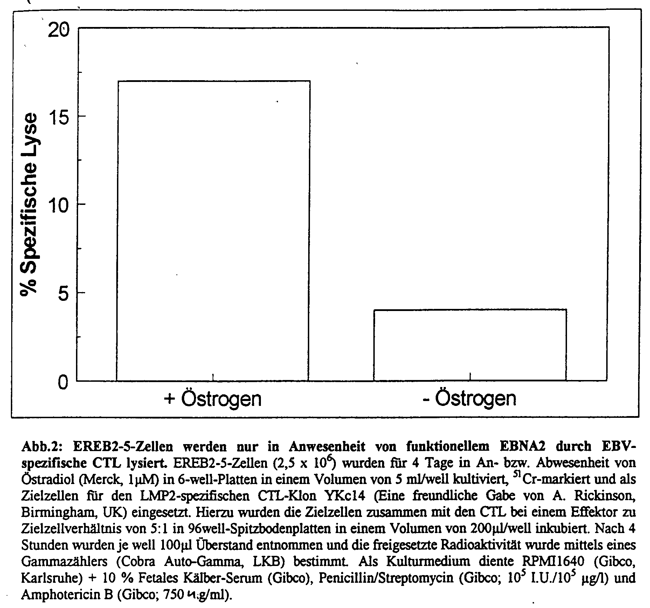

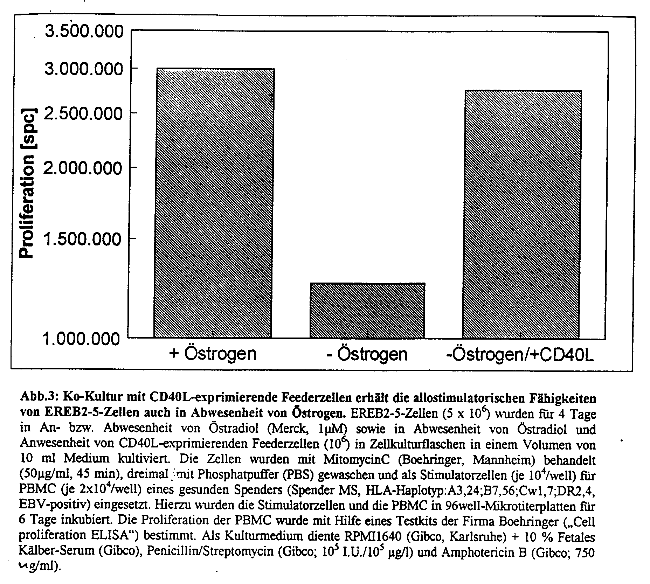

Die so erhaltenen LCL werden dann in Anwesenheit von Östrogen expandiert. Nach Östrogenentzug werden diese LCL ihre Proliferation einstellen (Abb.1) und gleichzeitig ihre Fähigkeit zur Stimulation von CTL mit Spezifität für EBNA2-regulierte EBV-kodierte Antigene verlieren (Abb.2). Da jedoch das Wunschantigen von einem auch nach Östrogenentzug aktiven Promotor exprimiert wird und die immunstimulatorische Kapazität dieser Zellen nach Ko-Kultur mit CD40L-exprierenden Feederzellen erhalten bleibt (Abb.3), sieht diese Ausführungsform des erfindungsgemäßen Verfahrens vor, die LCL nach Östrogenentzug in Anwesenheit solcher Feederzellen oder eines anderen CD40-Stimulus (löslicher Ligand, Antikörper) zu kultivieren. Nach einigen Tagen werden dann PBMC des Patienten zugegeben und die aktivierten T-Zellen mit den üblichen Standardmethoden kultiviert.

Unser Verfahren ist optimiert für die Generierung Klasse-I-restingierter CTL. Die heute bereits bekannten Verfahren, endogene Antigene in den Klasse-II-Weg der Antigenprozessierung einzuschleusen (Fusion des Antigens mit den sog. CLIP-Peptiden, Sekretion des Antigens), bieten jedoch die Möglichkeit, dieselben konditionalen LCL für die Generierung CD4-positiver T-Helferzellen (Tₕ) einzusetzen.

Das vorstehend beschriebene Verfahren ist deshalb zur Generierung von CTL optimiert, weil das gewünschte Antigen in erster Linie endogen exprimiert werden soll, so daß der Klasse I-Weg der Antigen-Prozessierung angesprochen wird. Um T-Helferzellen zu induzieren, ist die Einschleusung des Antigens in den Klasse II-Weg (auch als exogener Weg bezeichnet) nötig. Dies ist mit den heute bekannten Verfahren möglich über a) Fusion des Antigens an Signalpeptide, welche die sektretion des Antigens ermöglichen, so daß das Antigen aus der Zelle hinaus gelangt, von wo aus es dann in den exogenen Weg der Prozessierung gelangen kann; b) Fusion des Antigens an Peptide (sog. CLIP), welche aus der Klasse-II-assoziierten, invarianten Kette (Ii) stammen und welche die neu synthetisierten Klasse-II-Moleküle normalerweise in ein Kompartiment dirigieren, in dem die Assoziation mit antigenen Peptiden erfolgt. Hierdurch wird erreicht, daß das Antigen denselben Weg im Inneren der Zelle nimmt, den normalerweise leere Klasse-II-Moleküle nehmen; c) Zugeabe des Antigens von außen. Diese Methode setzt allerdings vorraus, daß man das Antigen oder antigene Peptide in halbwegs reiner form synthetisieren oder aus Naturprodukten isolieren kann.

### 2. Einsatz konditional EBV-immortalisierter LCL zur Reaktivierung einer bereits bestehenden T-Zell-Aktivierung nach Abschaltung von EBNA2

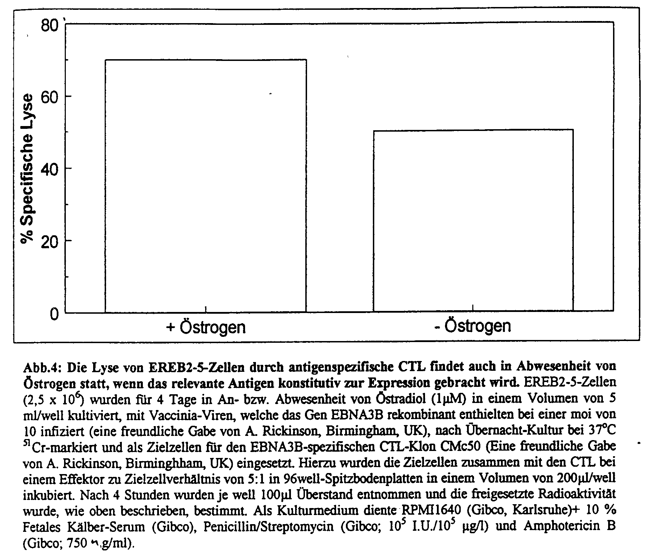

Diese Modifikation des oben beschriebenen Verfahrens kann angewendet werden, wenn bereits voraktivierte T-Zellen in einem Patienten vorliegen. Dies wird für zahlreiche Erkrankungen durch die hohe Frequenz, mit der *in-vitro* antigenspezifische T-Zellen aus solchen Patienten etablierbar sind, nahegelegt. Hierzu werden wie unter 1. konditionale LCL des Patienten etabliert. Anschließend wird diesen Zellen Östrogen entzogen und diese Zellen mit den PBMC des Patienten inkubiert. Unter diesen Umständen ist eine Aktivierung vorher geprimter antigenspezifischer CTL möglich (Abb. 4). Die gewonnenen CTL können dann mit Standardmethoden expandiert werden.

Dieses oben beschriebene Verfahren ist auch als Ausgestaltung des unter 1. beschriebenen Verfahrens einsetzbar, da zur Expansion der T-Zellen eine regelmäßige Restimulation der Zellen i.a. wünschenswert ist. Für die Restimulation der Zellen kann immer das unter 2. beschriebene Verfahren eingesetzt werden. Dies hat den großen Vorteil, daß kein CD40L benötigt wird.

### 3. Einsatz konditional EBV-immortalisierter LCL zur T-Zell-Aktivierung nach Abschaltung von EBNA2 in eine Zwei-Schritt-Verfahren

Diese Ausgestaltung des erfindungsgemäßen Verfahrens sieht vor, ausgehend von denselben konditional immortalisierten LCL diese in einem ersten Schritt in Anwesenheit von Östrogen mit den PBMC des Patienten zu inkubieren. Hierbei werden neben den gewünschten antigenspezifischen T-Zellen auch EBV-spezifische T-Zellen aktiviert. Anschließend werden für die Restimulation die LCL in Abwesenheit von Östrogen kultiviert. Hierbei werden die antigenspezifischen T-Zellen restimuliert (Abb.4), die EBV-spezifischen T-Zellen jedoch nicht (Abb. 2).

### 4. Einsatz allogener konditional EBV-immortalisierter LCL zur T-Zell-Aktivierung nach Abschaltung von EBNA2

### (a) Ein-Schritt-Verfahren

Diese Ausführungsform unterscheidet sich in einem wesentlichen Schritt von den Ausgestaltungen 1-3:

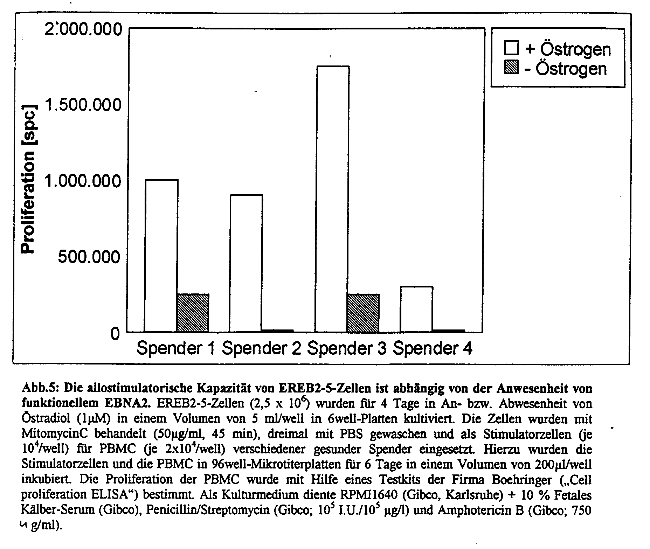

In einer Vorbereitungsphase werden konditional immortalisierte LCL von Spendern hergestellt, welche das gewünschte Antigen exprimieren. Diese LCL werden nach Abschalten von EBNA2 als APC für die PBMC eines Spenders eingesetzt, welcher syngen für ein entsprechendes MHC-Molekül mit dem Spender der LCL ist. Dies bedeutet, daß beispielsweise die PBMC eines HLA-A2-positiven Spenders mit den LCL eines ebenfalls A2-positiven Spenders inkubiert werden. Hierbei werden nur die bereits *in-vivo* geprimten A2-restringierten T-Zellen mit der gewünschten Spezifität expandiert. EBV-spezifische T-Zellen werden aus den bereits genannten Gründen nicht aktiviert. Der wesentliche Punkt hierbei ist, daß unter diesen Bedingungen allospezifische T-Zellen ebenfalls nicht aktiviert werden (Abb.5). Somit ist eine Aktivierung antigenspezifischer T-Zellen durch allogene LCL möglich. Die Bedeutung dieses Verfahrens liegt insbesondere darin, daß im Falle des therapeutischen Einsatzes von T-Zellen nicht erst LCL des Patienten individuell etabliert werden müssen, so daß für die Therapie wertvolle Zeit gewonnen wird.

### (b) Zwei-Schritt-Verfahren

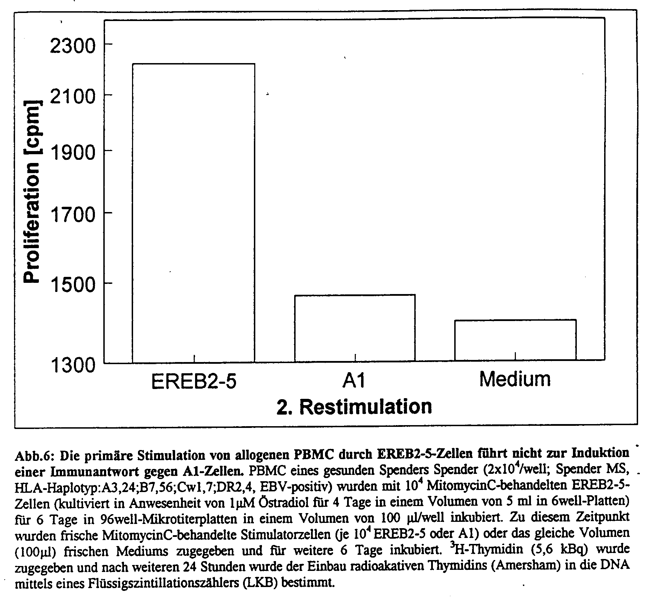

Dieses Verfahren bedient sich ebenfalls allogener konditional immortalisierter LCL. Da das unter 4a geschilderte Verfahren nur zur Aktivierung geprimter T-Zellen tauglich ist, sieht diese Modifikation des Verfahrens vor, in einem ersten Schritt eine Aktivierung der allogenen T-Zellen mit LCL in Anwesenheit der EBNA2-Funktion durchzuführen mit anschließender Restimulation der Zellen in Abwesenheit von Östrogen. Die zugrundeliegenden Mechanismen wurden bereits unter Punkt 3 erörtert. Wesentlich ist, daß neben der EBV-spezifischen T-Zellaktivierung die Allostimulation durch LCL nach Abschaltung von EBNA2 ebenfalls reduziert ist. T-Zellen, welche mit EREB2-5-Zellen als Alloantigen stimuliert wurden, zeigen keine Proliferation nach Restimulation mit A1, was zeigt, daß die fehlende Allostimulation durch LCL in Abwesenheit der EBNA2-Funktion nicht durch Priming der T-Zellen in Anwesenheit der EBNA2-Funktion überkommen werden kann (Abb.6).

Kommt es, wider Erwarten, unter diesen Bedingungen zu einer meßbaren Allostimulation, so bietet sich an, zur Restimulation konditional immortalisierte LCL einzusetzen, die das gleiche gewünschte Antigen exprimieren, aber von einer dritten Person stammen und (mit Außnahme der auch bei dem zu immunisierenden Patienten vorliegenden und zur Antigenpräsentation benötigten HLA-Moleküle) andere HLA-Allele exprimieren als die zur Primärstimulation verwendeten LCL. Die in der Primärstimulation aktivierten allospezifischen T-Zellen sollten somit in der Sekundärstimulation nicht aktiviert werden, da die relevanten Alloantigene auf den hierbei eingesetzten LCL fehlen.

Die Einsatzmöglichkeiten für T-Zell-Linien und -Klone, welche mit einem der erfindungsgemäßen Verfahren etabliert wurden, liegen einerseits in der immunologischen Grundlagenforschung, andererseits aber auch und gerade in dem adaptiven Transfer solcher Zellen in Patienten zur therapeutischen Modulation des Krankheitsverlaufs.

Die durch das erfindungsgemäß in vitro-Verfahren induzierten T-Zellen können wahlweise aufgereinigt und isoliert werden. Sie würden dann in den Patienten zur therapeutischen Anwendung, beispielsweise zur Tumortherapie oder zur Bekämpfung viraler Infektionen wie der nach Knochmarktransplantationen gefürchteten Zytomegalie-Virus-Infektion, adoptiv transferiert.

Erfindungsgemäß werden somit konditional immortalisierte, antigenpräsentierende Zellen zur Stimulierung immunkompetenter T-Zellen eingesetzt. Es wurde erfindungsgemäß überraschend festgestellt, daß nach Abschaltung der immortalisierenden Funktion, beispielsweise durch Abschaltung des EBNA2-Proteins, auch die immunstimulatorische Eigenschaft der antigenpräsentierenden Zellen für ungeprimte, also noch nicht zuvor für das Wunschantigen stimulierte T-Zellen nahezu vollständig verschwindet. Des weiteren wurde überraschend gefunden, daß eine Stimulation über die Interaktion von CD40 mit seinem Liganden die immunologische Erkennbarkeit der antigenpräsentierenden Zellen für ungeprimte T-Zellen wiederherstellbar ist.

### Allgemeine Angaben zur Methodik

Die Durchführung des ³H-Inkorporations-Assays und des ⁵¹Cr-Freisetzungs-Tests erfolgte nach Standardverfahren, wie Sie z.B. in [14,15] geschildert sind. Die Durchführung des "Cell proliferation ELISA" der Firma Boehringer erfolgte entsprechend den Angaben des Herstellers. Die Infektion von Zellen mit rekombinanten Vaccinia-Viren erfolgte bei einer moi (multiplicity of infection) von 10:1. Hierzu wurden die Zielzellen (500000) in 10 ml-Röhrchen abzentrifugiert und das Pellet mit dem Virushaltigen Überstand für 1,25 h inkubiert, wobei zwischendurch geschüttelt wurde. Anschließend wurden 2 ml Kulturmedium hinzugefügt und das Röhrchen über Nacht im Brutschrank gelagert und anschließend so weiterbehandelt wie dies für Zielzellen für den ⁵¹Cr-Freisetzungstest üblich ist.

Nachfolgend wird ein möglicher therapeutischer Einsatz der vorliegenden Erfindung beschrieben.

Ein Patient hat eine Chronisch-Myeloische Leukämie mit Expression des Bcr-Abl Fusionsproteins. Dieses Fusionsprotein stellt nicht nur das unseres Wissens nach für die Leukämieentstehung verantwortliche Agens dar, sondern ist auch ein tumorspezifisches Antigen, da die durch die Fusion entstandene Aminosäuresequenz nur in den Tumorzellen exprimiert wird. Die Existenz von T-Zellen, welche spezifisch diese Fusionsstelle erkennen, wurde bereits beschrieben.Vor der Knochenmarktransplantation werden vom Patienten und vom Spender periphere mononukleäre Zellen aus dem peripheren Blut entnommen und die B-Lymphozyten mit EBV konditional immortalisiert. Ausgehend von diesen Zellen werden zusätzlich Zellinien hergestellt, die. mit Bcr-Abl transfiziert wurden und das Bcr-Abl Fusionsprotein exprimieren. Die konditional immortalisierten, Bcr-Abl exprimierenden Zellen des Patienten werden nach Hormonentzug als antigenpräsentierende Zellen zur Stimulation der T-Zellen des Spenders eingesetzt. Die Spezifität der gewonnen T-Zellen wird mit Hilfe der konditional immortalisierten normalen sowie der Bcr-Abl exprimierenden Zellen des Patienten und des Spenders untersucht. T-Zellen, die das Bcr-Abl Fusionsprotein in Kombination mit HLA-Molekülen des Empfängers erkennen, werden in vitro expandiert und dem Patienten im Falle eines Rezidivs bzw. einer Blastenkrise reinfundiert. Durch die T-Zellen mit definierter Spezifität werden die Tumorzellen abgetötet und der Patient geheilt.

### Literatur:

[1] Die immunologischen Grundlagen sind den entsprechenden Lehrbüchern zu entnehmen. Falls nicht anders erwähnt, finden sich die wesentlichen Informationen beispielsweise in: Janeway, C. A., Jr. & P. Travers (1994). Immunobiology Oxford Blackwell Scientific Publications. Insbesondere Kapitel 4 und 7.
[2] Staerz, U. D., H. Karasuyama & A. M. Garner (1987). Cytotoxic T lymphocytes against a soluble Protein. Nature 329:449.
[3] Carbone, F. R. & M. J. Bevan (1989). Induction of ovalbumin-specific cytotoxic T cells by in-vivo peptide immunisation. J. Exp. Med. 169:603.
[4] Carbone, F. R., M. W. Moore, J. M. Sheil & M. J. Bevan (1988). Induction of cytotoxic T lymphocytes by primary in vitro stimulation with peptides. J. Exp. Med. 167:1767.
[5] Young, J. W. & K. Inaba (1996). Dendritic cells as adjuvants for class I major histocompatibility complex-restricted antitumor immunity. J.Exp. Med. 183:7.
[6] Aichele, P., K. Brduscha-Riem, R. M. Zinkernagel, H. Hengartner & H. Pircher (1995). T cell priming versus T cell torerance induced by synthetic peptides. J. Exp. Med. 182:261.
[7] Klein, G. (1994). Epstein-Barr virus strategy in normal and neoplastic B cells. Cell 77:791.
[8] Pecher, G. & O. J. Finn (1997). Induction of cellular immunity in chimpanzees to human tumor-associated antigen mucin by vaccination with MUC-1 cDNA transfected Epstein-Barr virus-immortalized autologous B cells. Proc. Natl. Acd. Sci. USA. 93:1699.
[9] Guilhot, s., P. Fowler, G. Portillo, R. F. Margolskee, C. Ferrari, A. Bertoletti & F. V. Chisari (1992). Hepatitis B Virus (HBV)-specific cytotoxic T-cell response in humans: production of target cells by stable expression of HBV-encoded proteins in immortalized human B-cell line. J. Virol. 66:2670.
[10] Penna, A., P. Fowler, A. Bertoletti, S. Guilhot, B. Moss, R. F. Margolskee, A. Cavalli, A. Valli, F. Fiaccadori, F. V. Chisari & C. Ferrari (1992). Hepatitis B vitus (HBV)-specific cytotoxic T-cell (CTL) response in humans: characterization of HLA class II-restricted CTLs that recognize endogenously synthesized HBV wenvelope antigens. J. Virol. 66 1193.
[11] Kempkes, B., D. Spitkovsky, D., P. Jansen-Dürt, J. W. Ellwart, E. Krenuner, H.-J. Delecluse, C. Rottenberger, G. W. Bornkamm & W. Hammerschrnidt (1995). B-cell proliferation and induction of early G1-regulating proteins by Epstein-Barr virus mutants conditional for EBNA2. EMBO J. 14:88.
[12] Polack, A., K. Hörtnagel, A. Pajic, B. Christoph, B. Baier, M. Falk, J. Mautner, C. Geltinger, G. W. Bornkamm & B. Kempkes (1996). c-myc activation renders proliferation of Epstein-Barr virus (EBV)-transformed cells independent of EBV nuclear antigen 2 and latent menmbran protein 1. Proc. Natl. Acad. Sci. USA 93:10411.
[13] Zimber-Strobl, U. B. Kempkes, G. Marschall, R. Zeidler, C. Van Kooten, J. Banchereau, G. W. Bomkamm und W. Hammerschmidt (1996). Epstein-Barr virus latent membran protein (LMPl) is not sufficient to maintain proliferation of B cells but both it and activated CD40 can prolong their survival. EMBO J. 15:7070.
[14] Siaege, M. S., T. Dick, R. Ertl, U. Jahnel, H. Nawrath, H.-G. Rammensee und A. B. Reske-Kurz. (1994). The antigen-self-presentation function of the cytotoxic T cell clone 10BK.1 depends on reciprocal peptide presentation. Immunology 81:333.
[15] Staege, M. S., T. Dick und A. B. Reske-Kunz. (1996). Functionally active T cell receptor/CD3 complexes are present at the surtace of cloned cytotoxic T cells without fluorescence-immunological detectability. Cell. Immunol. 171:62.
[16] Galibert L., Burdin N., des Saint-Vis B., Garrone P., Van Kooten C., Banchereau J. und Rousset F. (1996). CD40 and B cell antigen receptor dual triggering of resting B lymphocytes turns on a partial germinal center phenotype, J. Exp. Med. 183:77.
[17] N.M. Steven, A.M. Leese, N.E. Annels, S.P. Lee und A. Rickinso, 1996, Epitope fuocussing in the primary cytotoxic T cell response to Epstein-Barr Virus and is in relationship to T cell memory, J. Exp. Med. 184:1801.

## Patentansprüche

1. Verfahren zur Stimulation von T-Zellen mit gewünschter Antigenspezifität mit den nachfolgenden Schritten:
(a) Einführen von Immortalisierungs-Genen in antigenpräsentierende Zellen (APC) in solcher Weise, daß zumindest die Expression und/oder Funktion zumindest eines dieser Gene regulierbar ist, um konditional immortalisierte antigenpräsentierende Zellen zu erhalten;
(b) Einführen eines für das gewünschte Antigen kodierenden Gens in die im Schritt (a) erhaltenen immortalisierten Zellen in solcher Weise, daß das Antigen zumindest auch nach dem Stop der Expression und/oder der Aufhebung der Funktion zumindest eines der Immortalisierungsgene exprimierbar ist;
(c) Expansion der immortalisierten antigenpräsentierenden Zellen durch Expression der Immortalisierungsgene und/oder durch funktionelle Aktivierung der Immortalisierungsgene;
(d) Beendigung der Proliferation der immortalisierten antigenpräsentierenden Zellen durch Stop der Expression und/oder durch Aufhebung der Funktion zumindest eines der regulierbaren Immortalisierungsgene;
(e) fortgesetzte Expression des Antigens;
(f) Zugabe leukozytärer Zellen, die zumindest T-Zellen enthalten, und Kultivierung des Zellgemisches zur Stimulation der gegen das gewünschte Antigen gerichteten T-Zellen;
(g) wahlweise Aufreinigung und Isolierung der stimulierten T-Zellen.

2. Verfahren zur Stimulation von T-Zellen mit gewünschter Antigenspezifität mit den nachfolgenden Schritten:
(a) Einführen von Immortalisierungs-Genen in antigenpräsentierende Zellen (APC) in solcher Weise, daß zumindest die Expression und/oder Funktion zumindest eines dieser Gene regulierbar ist, um konditional immortalisierte antigenpräsentierende Zellen zu erhalten;
(b) Einführen eines für das gewünschte Antigen kodierenden Gens in die im Schritt (a) erhaltenen immortalisierten Zellen in solcher Weise, daß das Antigen zumindest auch nach dem Stop der Expression und/oder der Aufhebung der Funktion zumindest eines der Immortalisierungsgene exprimierbar ist;
(c) Expansion der immortalisierten antigenpräsentierenden Zellen durch Expression der Immortalisierungsgene und/oder durch funktionelle Aktivierung der Immortalisierungsgene;
(d) Zugabe leukozytärer Zellen, die zumindest T-Zellen enthalten, zu einem ersten Teil (Aliquot) der aus den Schritten (a) bis (c) erhaltenen immortalisierten, das gewünschte Antigen exprimierenden Zellen und Kultivierung des Zellgemisches zur Stimulation der gegen das gewünschte Antigen gerichteten T-Zellen;
(e) Beendigung der Proliferation des zweiten Teils der aus den Schritten (a) bis (c) erhaltenen, antigenpräsentierenden Zellen durch Stop der Expression und/oder durch Aufhebung der Funktion zumindest eines der für die Immortalisierung notwendigen regulierbaren Gene;
(f) Ko-Kultivierung der antigenpräsentierenden Zellen nach dem Stop der Expression und/oder der Aufhebung der Funktion zumindest eines der regulierbaren Immortalisierungsgene aus (e) mit den im Schritt (d) stimulierten T-Zellen zur Restimulation der gegen das gewünschte Antigen gerichteten T-Zellen; und
(g) wahlweise Aufreinigung und Isolierung der stimulierten T-Zellen.

3. Verfahren nach Anspruch 1,
dadurch gekennzeichnet, daß
im Schritt (a) APC aus einem Spender A zugegeben werden,
und im Schritt (f) allogene leukozytäre Zellen aus einem anderen Spender B eingesetzt werden, welcher syngen für mindestens ein entsprechendes, der Antigenpräsentation dienendes MHC-Molekül mit dem Spender A der APC ist.

4. Verfahren nach Anspruch 2,
dadurch gekennzeichnet, daß
im Schritt (a) APC aus einem Spender A zugegeben werden,
und im Schritt (d) allogene leukozytäre Zellen aus einem anderen Spender B eingesetzt werden, welcher syngen für mindestens ein entsprechendes, der Antigenpräsentation dienendes MHC-Molekül mit dem Spender A der APC ist.

5. Verfahren nach Anspruch 1,
dadurch gekennzeichnet, daß
die im Schritt (a) verwendeten APC vom gleichen Spender wie die leukozytären Zellen im Schritt (f) stammen.

6. Verfahren nach Anspruch 2,
dadurch gekennzeichnet, daß
die im Schritt (a) verwendeten APC vom gleichen Spender wie die leukozytären Zellen im Schritt (d) stammen.

7. Verfahren nach einem oder mehreren der vorhergehenden Ansprüche,
dadurch gekennzeichnet, daß
als Immortalisierungsgene Gene des Epstein-Barr Virus (EBV), Gene von Adenoviren oder Oncogene verwendet werden.

8. Verfahren nach einem oder mehreren der vorhergehenden Ansprüche,
dadurch gekennzeichnet, daß
als Vektoren zum Einführen des für das gewünschte Antigen kodierenden Gens in die immortalisierten antigenpräsentierenden Zellen von EBV, von Adenoviren, von Retroviren, von Foamyviren, Poxviren oder von SV40 abgeleitete Vektoren verwendet werden.

9. Verfahren nach Anspruch 8,
dadurch gekennzeichnet, daß
als von EBV-abgeleitete Vektoren mini-EBV-Vektoren verwendet werden und/oder als von Poxviren abgeleitete Vektoren Vacciniaviren verwendet werden.

10. Verfahren nach einem oder mehreren der vorhergehenden Ansprüche,
dadurch gekennzeichnet,
daß als das regulierbare Immortalisierungsgen des EBV das EBNA2-Gen, das EBNA3a-Gen, das EBNA3b-Gen, das EBNA3c-Gen oder das LMP-Gen verwendet wird.

11. Verfahren nach einem oder mehreren der vorhergehenden Ansprüche,
dadurch gekennzeichnet,
daß die regulierbaren Immortalisierungsgene durch Hormone oder Antibiotika regulierbar sind.

12. Verfahren nach Anspruch 11,
dadurch gekennzeichnet, daß
daß als Hormon ein Östrogen oder ein Androgen verwendet wird.

13. Verfahren nach einem oder mehreren der vorhergehenden Ansprüche,
dadurch gekennzeichnet,
daß die Schritte (a) und (b) gleichzeitig durchgeführt werden.

14. Verfahren nach Anspruch 1,
dadurch gekennzeichnet, daß
der Schritt (b) erst unmittelbar vor dem Schritt (e) durchgeführt wird.

15. Verfahren nach Anspruch 2,
dadurch gekennzeichnet, daß
der Schritt (b) erst unmittelbar vor dem Schritt (d) oder der Schritt (b) je unmittelbar vor den Schritten (d) und (f) erfolgt.

16. Verfahren nach einem oder mehreren der vorhergehenden Ansprüche,
dadurch gekennzeichnet,
daß das für das gewünschte Antigen kodierende Gen auf dem gleichen Vektor angeordnet wird, der die Immortalisierungsgene trägt, wobei zumindest eines dieser Immortalisierungsgene regulierbar ist.

17. Verfahren nach einem oder mehreren der vorhergehenden Ansprüche,
dadurch gekennzeichnet,
daß antigenspezifische, zytotoxische T-Zellen und/oder T-Helfer-Zellen und/oder T-Zellen mit Suppressor-Eigenschaften induziert werden.

18. Verfahren nach Anspruch 17,
dadurch gekennzeichnet, daß
als antigenspezifische zytotoxische T-Zellen Klasse I-MHC-restringierte zytotoxische T-Zellen und/oder als T-Helferzellen Klasse II-MHC-restringierte T-Helferzellen induziert werden.

19. Verfahren nach einem oder mehreren der vorhergehenden Ansprüche,
dadurch gekennzeichnet, daß
vor Zugabe der leukozytären Zellen die immunstimulatorischen Eigenschaften der das gewünschte Antigen exprimierenden, immortalisierten Zellen moduliert werden.

20. Verfahren nach Anspruch 19,
dadurch gekennzeichnet, daß
die Modulation durch Kultur in Anwesenheit eines CD40-Stimulus oder in Anwesenheit von Cytokinen erreicht wird.

21. Verfahren nach Anspruch 20,
dadurch gekennzeichnet,
daß der CD40-Stimulus durch Zugabe von CD40-exprimierenden Feeder-Zellen und Co-Kultivierung der immortalisierten antigenpräsentierenden Zellen und der Feeder-Zellen erhalten wird oder
daß der CD40-Stimulus durch Zugabe eines löslichen CD40-Liganden oder eines anti-CD40-Antikörpers erfolgt.

22. Verfahren nach einem oder mehreren der vorhergehenden Ansprüche,
dadurch gekennzeichnet,
daß als antigenpräsentierende Zellen B-Zellen, Makrophagen, dendritische Zellen und/oder Fibroblasten verwendet werden.

23. Verfahren nach einem oder mehreren der vorhergehenden Ansprüche,
dadurch gekennzeichnet, daß
vor Zugabe der leukozytären Zellen das Wachstum der immortalisierten, antigenpräsentierenden Zellen dauerhaft unterdrückt wird.

24. Verfahren nach Anspruch 23,
dadurch gekennzeichnet, daß
durch Zugabe von Mitomycin C oder durch Bestrahlung die Unterdrückung erfolgt.

25. Verfahren nach einem oder mehreren der vorhergehenden Ansprüche,
dadurch gekennzeichnet, daß
T-Zellen aus Säugern stimuliert werden, bevorzugt aus Mensch und Nagetieren.
